# EUROPEAN PATENT APPLICATION

(11) **EP 4 850 704 A1**
(43) Date of publication of application: **09.12.2026**
(21) Application number: 24920883.6
(22) Date of filing: 31.01.2024
(51) Int. Cl.: A61L 27/20, A61L 27/26, A61L 27/52, A61L 27/12, A61L 27/46, C08L 5/08, C08L 1/28, A61K 8/73

(54) **HOMOGENEOUS GEL COMPOSITION FOR DERMIS FILLING, AND PREPARATION METHOD THEREFOR AND USE THEREOF**

(71) Applicant: OSDERMA Hong Kong Limited, Hong Kong (HK)
(72) Inventor: ZHAO, Doudou, Shanghai 201201 (CN); MA, Chi, Shanghai 201201 (CN); ZHENG, Shijie, Shanghai 201201 (CN); HUANG, Xinfan, Shanghai 201201 (CN)
(74) Representative: Lavoix
(86) International application number: PCT/CN2024/074950
(87) International publication number: WO 2025/160811

(57) **Abstract**

Disclosed in the present invention are a homogeneous gel composition for dermis filling, and a preparation method therefor and the use thereof. The homogeneous gel composition comprises: (Y1) second biocompatible solid particles; and (Y2) a gel material. The gel material comprises the following components: (Z1) first biocompatible solid particles; (Z2) hyaluronic acid; and (Z3) a cellulose-based polysaccharide polymer, wherein the gel material is in a solid powder or gel form. The homogeneous gel composition prepared in the present invention comprises high- and low-association-degree biocompatible solid particles, such that the biocompatible solid particles are gradually released in a matrix, and the biocompatible solid particles can stably exist in a composite system for a long time.

## Description

### Technical field

The present invention relates to the technical field of medical aesthetics, and specifically provides a homogeneous gel composition for dermal filling, a preparation method therefor, and applications thereof.

### Background

Currently, people (especially women) often seek repair or surgery for changes in the skin caused by aging, disease, trauma, or other reasons. Dermal filling and botulinum toxin wrinkle removal are two of the most popular options. Dermal filling can effectively eliminate wrinkles and deep lines, thereby providing filling effects to smooth facial contours. Dermal filling has a history of over 40 years. Historically, different methods have been attempted to improve facial aesthetics and help people look younger.

Many dermal fillers have been introduced, with varying clinical effects. For example, soft tissue fillers can be classified into temporary fillers (autologous fat, collagen, hyaluronic acid, etc.), semi-permanent fillers (composite materials loaded with calcium phosphate particles or polylactic acid (PLA)), and permanent fillers (silicone, polymethylmethacrylate (PMMA), polytetrafluoroethylene (PTFE), etc.). However, the effects of existing dermal fillers are often unsatisfactory, and they possess various drawbacks, such as poor injectability, low biocompatibility, low stability, and difficulty in loading high contents of solid particles like hydroxyapatite.

Therefore, there is an urgent need in the art to provide a gel composition with high injectability, high biocompatibility, high stability, and the capability to load high contents of solid particles.

### Summary of the invention

The purpose of the present invention is to provide a gel composition with high injectability, high biocompatibility, high stability, and the capability to load high contents of solid particles, as well as a preparation method therefor and applications thereof (such as use as a dermal filler or an implant composition for bone sculpting).

In the first aspect of the present invention, a gel material is provided, which comprises following components: (Z1) the first biocompatible solid particles; (Z2) hyaluronic acid (HA); and (Z3) a cellulose-based polysaccharide polymer; wherein the gel material is in a solid powdery form or a gel form.

In another preferred embodiment, the gel material in a solid powdery form is formed by drying and crushing the gel material in a gel form.

In another preferred embodiment, the gel material in a gel form is obtained by reconstituting the gel material in a solid powdery form with water or an aqueous buffer solution into a gel material in a gel form.

In another preferred embodiment, the gel material in a gel form possesses physicochemical characteristics selected from the group consisting of:
(a) the gel material in a gel form comprises highly associated biocompatible solid particles;
(b) the gel material in a gel form has a pH of 6-8;
(c) the gel material in a gel form has a water content of 75%-95%.

In another preferred embodiment, the hyaluronic acid and the cellulose-based polysaccharide polymer in the gel material form a mixture, and the hyaluronic acid undergoes crosslinking to form a gel composite support skeleton.

In another preferred embodiment, component (Z1) the first biocompatible solid particles and the gel composite support skeleton form a composite structure.

In another preferred embodiment, the weight ratio of hyaluronic acid to the cellulose polysaccharide polymer is: 1:0.5~1:5, preferably 1:1~1:4, more preferably 1:1.5~1:2.5.

In another preferred embodiment, the weight ratio of hyaluronic acid to the first biocompatible solid particles is: 1:1~1:4, preferably 1:2~1:3.

In another preferred embodiment, the hyaluronic acid has a molecular weight of 80~200 wDa.

In another preferred embodiment, the components Z1, Z2, and Z3 account for 60%~100%, preferably 70%~100%, more preferably 80%~100% of the dry weight of the gel material.

In another preferred embodiment, the first biocompatible solid particles are selected from the group consisting of: calcium phosphate salt particles, silicate particles, calcium sulfate salt particles, ceramic particles, biological bone matrix particles, organic solid particles, or combinations thereof.

In another preferred embodiment, the calcium phosphate salt particles are selected from the group consisting of: hydroxyapatite (HAP), β-tricalcium phosphate (β-TCP), α-tricalcium phosphate (α-TCP), tetracalcium phosphate (TTCP), or combinations thereof.

In another preferred embodiment, the silicate particles are selected from the group consisting of: bioglass, calcium silicate, sodium silicate, or combinations thereof.

In another preferred embodiment, the calcium sulfate salt particles are selected from hydrated calcium sulfate.

In another preferred embodiment, the organic solid particles are selected from the group consisting of: PMMA, PLA, Poly (ε-caprolactone) (PCL), poly(lactic-co-glycolic acid) (PLGA), or combinations thereof.

In another preferred embodiment, the first biocompatible solid particles are hydroxyapatite.

In another preferred embodiment, the first biocompatible solid particles are hollow hydroxyapatite microspheres.

In another preferred embodiment, the hollow hydroxyapatite microspheres are nano-cluster hollow hydroxyapatite microspheres.

In another preferred embodiment, the first biocompatible solid particles have a particle size ≤500 µm; preferably a particle size of 10~500 µm, more preferably a particle size of 25~250 µm, most preferably a particle size of 20~150 µm.

In another preferred embodiment, the first biocompatible solid particles have a particle size of 20~50 µm.

In another preferred embodiment, the cellulose-based polysaccharide polymer is selected from the group consisting of: hydroxypropyl methylcellulose, carboxymethyl cellulose, hydroxyethyl cellulose, ethyl hydroxyethyl cellulose, sodium carboxymethyl cellulose, carboxymethyl hydroxyethyl cellulose, hydroxypropyl hydroxyethyl cellulose, methyl cellulose, methyl hydroxymethyl cellulose, methyl hydroxyethyl cellulose, carboxymethyl methyl cellulose, or combinations thereof.

In another preferred embodiment, the cellulose-based polysaccharide polymer is also selected from the group consisting of: cellulose-based polysaccharide derivatives, modified cellulose-based polysaccharide derivatives, or combinations thereof.

In another preferred embodiment, the cellulose-based polysaccharide polymer is hydroxypropyl methylcellulose.

In the second aspect of the present invention, a preparation method for the gel material is provided, comprising following steps:
(S1) providing the first mixture, the first mixture comprising: hyaluronic acid, a cellulose polysaccharide polymer, and the first biocompatible solid particles;
(S2) in the presence of a crosslinking agent, subjecting the hyaluronic acid in the first mixture to a crosslinking reaction, thereby forming a crosslinked mixture with the cellulose-based polysaccharide polymer.

In another preferred embodiment, the preparation method further comprises:
(S3) dropwise adding the mixture into an acetone solution to wash away residual crosslinking agent, then drying to obtain the gel material in a solid powdery form; dissolving the gel material in a solid powdery form in water and/or an aqueous buffer solution to obtain the reconstituted gel material in a gel form.

In another preferred embodiment, step (S2) further comprises treating the crosslinked mixture as follows:
(i) adjusting the pH of the crosslinked mixture to acidic to terminate the crosslinking reaction, eluting the crosslinking agent and solidifying,
(ii) washing and drying to obtain the gel material in a solid powdery form,
(iii) dissolving the gel material in a solid powdery form in water and/or an aqueous buffer solution to obtain the gel material in a gel form.

In another preferred embodiment, the crosslinking reaction is carried out under alkaline conditions.

In another preferred embodiment, the alkaline conditions are a pH of 11~13.

In another preferred embodiment, the acidic environment is adjusting the pH to 4~6.8.

In another preferred embodiment, the crosslinking agent is selected from the group consisting of: 1,4-Butanediol diglycidyl ether, poly(ethylene glycol) diglycidyl ether, 1,6-Hexanediol diglycidyl ether, polypropylene glycol diglycidyl ether, polytetramethylene glycol diglycidyl ether, polyglycerol polyglycidyl ether, neopentyl glycol diglycidyl ether, ethylene glycol diglycidyl ether, or combinations thereof.

In another preferred embodiment, the crosslinking agent is 1,4-Butanediol diglycidyl ether (BDDE).

In the third aspect of the present invention, an injectable homogeneous gel composition is provided, the homogeneous gel composition comprising:
(Y1) the second biocompatible solid particles; and
(Y2) the gel material according to the first aspect of the present invention.

In another preferred embodiment, the second biocompatible solid particles and the gel material in a gel form according to the first aspect of the present invention are physically mixed to form a low association degree, thereby obtaining a homogeneous gel composition comprising biocompatible solid particles of both high association degree and low association degree.

In another preferred embodiment, (W1+W2)/W0 = 5:1-20:1; preferably 8:1-15:1; wherein W1 is the weight of the first biocompatible solid particles, W2 is the weight of the second biocompatible solid particles, and W0 is the weight of the reconstituted gel material (excluding the biocompatible solid particles).

In another preferred embodiment, the second biocompatible solid particles are the same as or different from the first biocompatible solid particles.

In another preferred embodiment, the particle sizes of the second biocompatible solid particles can be the same or different.

In another preferred embodiment, the homogeneous gel composition has one or more characteristics selected from the group consisting of:
(a) the homogeneous gel composition comprises biocompatible solid particles of both high association degree and low association degree;
(b) the biocompatible solid particles of the homogeneous gel composition account for 2%-95%, preferably 3%-80%, more preferably 4%-60%, most preferably 5%-30% of the dry weight of the homogeneous gel composition;
(c) the hyaluronic acid content in the homogeneous gel composition is 0.5%-4% w/w (5-40 mg/g), calculated based on the weight of the homogeneous gel composition excluding the biocompatible solid particles;
(d) the cellulose-based polysaccharide polymer content in the homogeneous gel composition is 0.2%-8% w/w (2-80 mg/g), calculated based on the weight of the homogeneous gel composition excluding the biocompatible solid particles.

In the fourth aspect of the present invention, a method for preparing the homogeneous gel composition according to the third aspect of the present invention is provided, comprising following steps:
(S3) providing the second mixture, the second mixture comprising: the gel material in a gel form according to the first aspect of the present invention and the second biocompatible solid particles, or the gel material in a solid powdery form, the second biocompatible solid particles, and water or an aqueous buffer solution;
(S4) mixing the second mixture uniformly to form the homogeneous gel composition.

In another preferred embodiment, when the gel material used in step (S3) is in a solid powdery form, it is necessary to add a buffer solution for reconstitution to obtain the gel material in a gel form.

In another preferred embodiment, the gel material is reconstituted with a PBS buffer solution.

In another preferred embodiment, the method further comprises following steps: degassing, packaging, and sterilizing the gel composite from step (S3) to form the homogeneous gel composition.

In the fifth aspect of the present invention, a use of the homogeneous gel composition according to the third aspect of the present invention is provided, the use of the homogeneous gel composition comprising: dermal filling, and bone sculpting.

In the fourth aspect of the present invention, a kit is provided, the kit comprising following components: the homogeneous gel material according to the first aspect of the present invention, or the homogeneous gel composition according to the third aspect of the present invention.

In another preferred embodiment, the kit further comprises: a pre-filled syringe, instructions for use, and spare injection needles.

It should be understood that within the scope of the present invention, the various technical features of the present invention described above and the various technical features specifically described below (e.g., in the embodiments) can be combined with each other, thereby constituting new or preferred technical solutions. Due to space limitations, each and every combination is not exhaustively described herein.

### Description of the drawings

Figure 1 shows the solid powder of the gel after freeze-drying.
Figure 2 shows the injectability results of gels prepared with HA and HPMC at different ratios.
Figure 3 shows the stability of the homogeneous composition with and without HPMC.
Figure 4 shows the preparation flowchart of the homogeneous gel composition.
Figure 5 shows the hydroxyapatite sedimentation in Examples 1-5, wherein A in Figure 5 shows the hydroxyapatite sedimentation in Example 1, B in Figure 5 shows the hydroxyapatite sedimentation in Example 2, C in Figure 5 shows the hydroxyapatite sedimentation in Example 3, D in Figure 5 shows the hydroxyapatite sedimentation in Example 4, and E in Figure 5 shows the hydroxyapatite sedimentation in Example 5.

### Detailed Description

Through extensive and in-depth research, the inventors of the present invention, for the first time, have unexpectedly developed a composite gel material and a corresponding injectable homogeneous gel composition, which uses crosslinked hyaluronic acid and a cellulose-based polysaccharide polymer as the gel carrier (or gel support skeleton) and incorporates highly loaded biocompatible solid particles. The gel material and the injectable homogeneous gel composition of the present invention contain a composite structure composed of specific components, wherein component (Z2) hyaluronic acid undergoes crosslinking in the presence of component (Z1) first biocompatible solid particles and component (Z3) cellulose-based polysaccharide polymer, endowing the first biocompatible solid particles with a high association degree and high loading capacity. In the injectable homogeneous gel composition of the present invention, the gel material of the present invention and the second biocompatible solid particles are combined through adsorption and physical mixing, thereby endowing the second biocompatible solid particles with a low association degree, ultimately forming a homogeneous gel composition characterized by both high and low association degrees. Tests have shown that the homogeneous gel composition of the present invention contains at least two types of biocompatible solid particles with different association degrees, enabling the progressive release of the biocompatible solid particles from the matrix and allowing them to stably exist in the composite system over the long term. The present invention is completed on this basis.

### Terms

Unless otherwise defined, all technical and scientific terms used herein have the same meaning as commonly understood by one of ordinary skill in the art to which this invention belongs.

As used herein, the term "about", when referring to a specifically listed numerical value, means that the value may vary from the listed value by no more than 1%. For example, as used herein, the expression "about 100" includes 99 and 101 and all values in between (e.g., 99.1, 99.2, 99.3, 99.4, etc.).

As used herein, the terms "comprising" or "including" can be open-ended, semi-closed, or closed. In other words, the terms also include "consisting essentially of" or "consisting of".

As used herein, the terms "HAP" and "hydroxyapatite", "nu-HAP" and "nano-cluster hollow hydroxyapatite microspheres" are used interchangeably.

As used herein, the terms "BDDE" and "1,4-butanediol diglycidyl ether" are used interchangeably.

As used herein, the terms "tightly associated" or "high association degree" and "loosely associated" or "low association degree" regarding the association of gel and hydroxyapatite shall be interpreted as relating to regions of the gel where the biocompatible solid particles have different degrees (high and low, respectively) of association with the gel.

### Homogeneous Gel Composition

In the present invention, the terms "homogeneous gel composition", "homogeneous gel", "homogeneous hydroxyapatite-hyaluronic acid gel", "homogeneous hydroxyapatite-hyaluronic acid-hydroxypropyl methyl cellulose gel", "homogeneous hydroxyapatite-hyaluronic acid-hydroxypropyl methyl cellulose gel with different association degrees", or similar terms, have the same meaning emphasizing homogeneity.

### Hyaluronic Acid

Hyaluronic acid, also known as hyaluronan, abbreviated as HA, is an acidic mucopolysaccharide. It is a common component in injectable filler formulations and is used in several cosmetic procedures, particularly for wrinkle filling. Due to rapid enzymatic degradation and hydrolysis, the *in vivo* stability of natural hyaluronic acid is poor, which is insufficient for the long-term stable existence of hydroxyapatite particles.

In the present invention, suitable hyaluronic acid has a molecular weight of 50-200 wDa. It should be understood that hyaluronic acid applicable to the present invention includes both unmodified and modified hyaluronic acid. For example, representative modifications include (but are not limited to): chemical crosslinking, ionic modification, esterification, etc.

Studies in the present invention have shown that when solely using crosslinked hyaluronic acid as the carrier, its injectability is significantly affected.

### Cellulose-Based Polysaccharide Polymer

Cellulose-based polysaccharide polymers possess good biocompatibility, biodegradability, and excellent mechanical stability, and are widely used in biomedical applications (such as drug sustained release, wound healing, tissue engineering scaffolds, etc.). Cellulose-based polysaccharide polymers have sufficiently large pore structures and three-dimensional spaces, with strong water absorption and retention functions, providing a space for the survival and storage of nutrients for cells *in vivo*. Furthermore, due to their specific structure and properties, they have become the preferred materials for injectable formulations, used for filling tissue defect sites.

In the present invention, cellulose-based polysaccharide polymers include natural cellulose or modified cellulose. Representative cellulose-based polysaccharide polymers include (but are not limited to): hydroxypropyl methyl cellulose, carboxymethyl cellulose, hydroxyethyl cellulose, ethyl hydroxyethyl cellulose, sodium carboxymethyl cellulose, carboxyethyl hydroxyethyl cellulose, hydroxypropyl hydroxyethyl cellulose, methyl cellulose, methyl hydroxymethyl cellulose, methyl hydroxyethyl cellulose, carboxymethyl methyl cellulose, or combinations thereof.

Particularly preferred cellulose-based polysaccharide polymers include: Hydroxypropyl methyl cellulose (HPMC), carboxymethyl cellulose, or combinations thereof.

### Biocompatible Solid Particles

"Biocompatible solid particles" in the present invention refer to active solid particles or solid particles with good biocompatibility that can play a role in certain biological processes.

Preferably, the biocompatible solid particles are selected from the group consisting of: calcium phosphate salt particles, silicate particles, calcium sulfate salt particles, ceramic particles, biological bone matrix particles, organic solid particles, or combinations thereof.

Preferably, the calcium phosphate salt particles are selected from the group consisting of: HAP, β-TCP, α-TCP, TTCP, or combinations thereof.

Preferably, the silicate particles are selected from the group consisting of: bioglass, calcium silicate, sodium silicate, or combinations thereof.

Preferably, the calcium sulfate salt particles are selected from hydrated calcium sulfate.

Preferably, the organic solid particles are selected from the group consisting of: PMMA, PCL, PLA, PLGA, or combinations thereof.

Preferably, the biocompatible solid particles are hydroxyapatite.

Preferably, the biocompatible solid particles are hollow hydroxyapatite microspheres.

Preferably, the hollow hydroxyapatite microspheres are nano-cluster hollow hydroxyapatite microspheres.

### Hydroxyapatite (HAP)

Hydroxyapatite (HAP), molecular formula: (Ca₁₀(PO₄)₆(OH)₂), is the main inorganic component of human bones. The microscopic morphology of natural HAP in the human body is needle-rod-shaped nanocrystals. HAP has bidirectional regulatory biological functions. HAP can form chemical bonds with adsorbates or undergo metal ion exchange; therefore, it has a strong adsorption capacity for proteins and various growth factors, can promote the enrichment of local autologous growth factors, and promote tissue repair and regeneration, thus exhibiting excellent biocompatibility and bioactivity. The degradation products of HAP are calcium ions, phosphate ions, and water. While adsorbing, the released calcium and phosphate ions exert other physiological functions.

### Nano-Cluster Hollow Hydroxyapatite Microspheres (nu-HAP)

Nano-cluster hollow hydroxyapatite microspheres (nu-HAP) are constructed based on needle-rod-shaped hydroxyapatite at the nanoscale, forming a cluster-like stacking morphology, and are then prepared into hollow microspheres. Their larger specific surface area provides more attachment sites for proteins and bioactive factors, and their larger particle size contributes to a better dermal filling effect, enabling the hollow hydroxyapatite microspheres to fully utilize the bidirectional regulatory function of HAP while meeting clinical requirements.

### Organic Solid Particles - PMMA Microspheres

Polymethyl methacrylate (PMMA) microspheres can continuously stimulate the growth of subcutaneous collagen and other subcutaneous tissues. After collagen products containing PMMA microspheres are injected into the deep dermis, the injected collagen will gradually be absorbed by the human body over several months. The PMMA microspheres continuously stimulate collagen regeneration. After 1-3 months, the autologously produced collagen produced replaces the original collagen. As long as the amount of collagen under the skin remains stable, the skin can be kept free of wrinkles for a long time, serving the purpose of filling facial wrinkles and anti-aging.

### Bioactive Glass

Bioactive glass (BAG) is a type of material that can repair, replace, and regenerate body tissues, and has the ability to form bonds between tissue and the material. It is a silicate glass composed of basic components such as SiO₂, Na₂O, CaO, and P₂O₅. The degradation products of bioactive glass can promote the generation of growth factors, enhance cell proliferation, increase the gene expression of osteoblasts and the growth of bone tissue, and are widely used in the field of bone sculpting.

### Gel Material of the Present Invention

The gel material according to the present invention contains following components: (Z1) the first biocompatible solid particles; (Z2) hyaluronic acid; and (Z3) a cellulose-based polysaccharide polymer; wherein the gel material is in a solid powdery form or a gel form.

In another preferred embodiment, the gel material in a solid powdery form is formed by drying and crushing a gel material in a gel form.

In another preferred embodiment, the gel material in a solid powdery form is reconstituted into a gel material in a gel form by adding water or an aqueous buffer solution.

In another preferred embodiment, the gel material in a gel form has physicochemical characteristics selected from the group consisting of:
(a) the gel material in a gel form comprises highly associated biocompatible solid particles;
(b) the gel material in a gel form has a pH of 6-8;
(c) the gel material in a gel form has a water content of 75%-95%.

In another preferred embodiment, the mixture of crosslinked component (Z2) hyaluronic acid and component (Z3) cellulose-based polysaccharide polymer serves as a gel support skeleton for component (Z1) the first biocompatible solid particles.

In another preferred embodiment, component (Z1) the first biocompatible solid particles and the gel support skeleton form a composite structure.

In another preferred embodiment, the weight ratio of hyaluronic acid to the cellulose polysaccharide polymer is: 1:0.5~1:5, preferably 1:1~1:4, more preferably 1:1.5~1:2.5.

In another preferred embodiment, the weight ratio of hyaluronic acid to the first biocompatible solid particles is: 1:1~1:4, preferably 1:2~1:3.

In another preferred embodiment, the hyaluronic acid has a molecular weight of 80~200 wDa.

In another preferred embodiment, components Z1, Z2, and Z3 account for 60%~100%, preferably 70%~100%, more preferably 80%~100% of the dry weight of the gel material.

### Preparation of the Gel Material of the Present Invention

The preparation method for the gel material comprises following steps:
(S1) providing the first mixture, the first mixture comprising: hyaluronic acid, a cellulose polysaccharide polymer, and the first biocompatible solid particles;
(S2) in the presence of a crosslinking agent, causing a crosslinking reaction of the hyaluronic acid in the first mixture, thereby forming a crosslinked mixture.

In another preferred embodiment, step (S2) further comprises treating the crosslinked mixture as follows:
(i) adjusting the pH of the crosslinked mixture to acidic to terminate the crosslinking reaction, eluting the crosslinking agent and solidifying,
(ii) washing and drying to obtain the gel material in a solid powdery.
(iii) dissolving the gel material in a solid powdery in water and/or an aqueous buffer solution to obtain the gel material in a gel form.

In another preferred embodiment, the crosslinking reaction is carried out under alkaline conditions.

In another preferred embodiment, the alkaline conditions are a pH of 11~13.

In another preferred embodiment, the acidic environment is adjusting the pH to 4~6.8.

In another preferred embodiment, the crosslinking agent is selected from the group consisting of: 1,4-butanediol diglycidyl ether, poly(ethylene glycol) diglycidyl ether, 1,6-hexanediol diglycidyl ether, polypropylene glycol diglycidyl ether, polytetramethylene glycol diglycidyl ether, polyglycerol polyglycidyl ether, neopentyl glycol diglycidyl ether, ethylene glycol diglycidyl ether, or combinations thereof.

In another preferred embodiment, the crosslinking agent is 1,4-butanediol diglycidyl ether (BDDE).

### Injectable Homogeneous Gel Composition of the Present Invention

The homogeneous gel composition comprises: (Y1) the second biocompatible solid particles; and (Y2) said gel material.

In another preferred embodiment, the second biocompatible solid particles and the gel material in a gel form are combined through adsorption and physical mixing, thereby forming a homogeneous gel composition comprising highly associated and lowly associated biocompatible solid particles.

In another preferred embodiment, (W1+W2)/W0 = 5:1-20:1; preferably 8:1-15:1; wherein W1 is the weight of the first biocompatible solid particles, W2 is the weight of the second biocompatible solid particles, and W0 is the weight of the reconstituted gel material (excluding biocompatible solid particles).

In another preferred embodiment, the second biocompatible solid particles are the same as or different from the first biocompatible solid particles.

In another preferred embodiment, the particle sizes of the second biocompatible solid particles can be the same or different.

In another preferred embodiment, the homogeneous gel composition has one or more characteristics selected from the group consisting of:
(a) the homogeneous gel composition comprises highly associated and lowly associated biocompatible solid particles;
(b) the biocompatible solid particles account for 2%-95%, preferably 3%-80%, more preferably 4%-60%, most preferably 5%-30% of the dry weight of the homogeneous gel composition.
(c) the hyaluronic acid content in the homogeneous gel composition is 0.5%-4% w/w (5-40 mg/g), calculated based on the weight of the homogeneous gel composition excluding biocompatible solid particles;
(d) the cellulose-based polysaccharide polymer content in the homogeneous gel composition is 0.2%-8% w/w (2-80 mg/g), calculated based on the weight of the homogeneous gel composition excluding biocompatible solid particles.

### Preparation of the Homogeneous Gel Composition of the Present Invention

The preparation method for the homogeneous gel composition comprises following steps:
(S3) providing the second mixture, the second mixture comprising: the gel material in a gel form and the second biocompatible solid particles, or the gel material in a solid powdery form, the second biocompatible solid particles, and water or an aqueous buffer solution;
(S4) mixing the second mixture uniformly to form the homogeneous gel composition.

In another preferred embodiment, when the gel material used in step (S3) is in the solid powdery form, it is necessary to add a buffer solution for reconstitution to obtain the gel-form gel material.

In another preferred embodiment, the gel material is reconstituted with a PBS buffer solution.

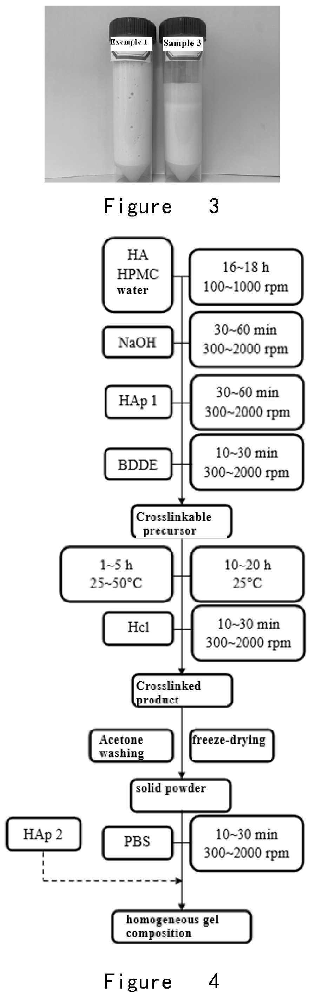

In another preferred embodiment, the method further comprises following steps: degassing, packaging, and sterilizing the gel composite from step (S3) to form the homogeneous gel composition. The preparation flowchart is shown in Figure 4.

### Uses of the Homogeneous Gel Composition

The uses of the homogeneous gel composition comprise: dermal filling, bone sculpting.

### Kit

The kit comprises following components: the homogeneous gel material or the homogeneous gel composition.

In another preferred embodiment, the kit further comprises: a pre-filled syringe, instructions, spare injection needles.

### Compared with the prior art, the beneficial effects of the present invention are as follows:

(a) The present invention uses crosslinked hyaluronic acid and a cellulose-based polysaccharide polymer as the carrier for biocompatible solid particles. It leverages the properties of crosslinked hyaluronic acid and the stability of the cellulose-based polysaccharide polymer to provide a long-term stable support for the solid particles.
(b) The present invention obtains a homogeneous gel containing biocompatible solid particles with different association degrees by adding the biocompatible solid particles before and after the crosslinking reaction. This enables the gradual release of the biocompatible solid particles from the matrix, allowing them to stably exist in the composite system over a long term.
(c) The hydroxypropyl methylcellulose used in this method is readily available, safe, and is a commonly used pharmaceutical excipient. This reaction has the advantages of mild reaction conditions and simple operational steps.
(d) In this method, the hyaluronic acid crosslinking agent BDDE is readily available and easily to be removed. The crosslinking reaction conditions are mild, facilitating easy control over the crosslinking degree of the hyaluronic acid.
(e) The nano-cluster hollow hydroxyapatite microspheres used in this method have a higher specific surface area, providing more attachment sites for active proteins, and can effectively promote the adsorption and regeneration of collagen.

The following, in combination with specific implementation examples, further elaborates on the present invention. It should be understood that these examples are only for illustrating the present invention and are not intended to limit the scope of the invention. In the following examples, experimental methods without specified conditions are generally carried out under conventional conditions or according to the conditions suggested by the manufacturer. Unless otherwise stated, percentages and parts are by weight.

### Example 1. Preparation of a Tightly Associated Biocompatible Solid Particle Gel

2 g of hyaluronic acid and 1 g of hydroxypropyl methylcellulose were added to 27 g of water, and mixed at 500 rpm at room temperature until homogeneous. Subsequently, 1 g of sodium hydroxide (1 M) solution was added to the mixture to adjust the pH > 12, and mixed further at 500 rpm for 30 min. 5.26 g of hollow hydroxyapatite microspheres with a particle size of 20~50 µm was added to the mixture, and mixed again at 300 rpm for 30 min. Subsequently, 0.2 g of 1,4-butanediol diglycidyl ether (BDDE) was added, and the mixture was homogenized at 300 rpm for another 30 min. The homogenized mixture was placed in a 45°C oven for 3 h, and then let it stand at 25°C for 12 h. Then, 5 g of hydrochloric acid (0.5 M) was added to adjust the pH < 7 to terminate the crosslinking reaction. The mixture was stirred at 300 rpm for 10 minutes to obtain a gel-like, slightly acidic composite gel in a state between solid and liquid, containing hydroxyapatite hollow microspheres.

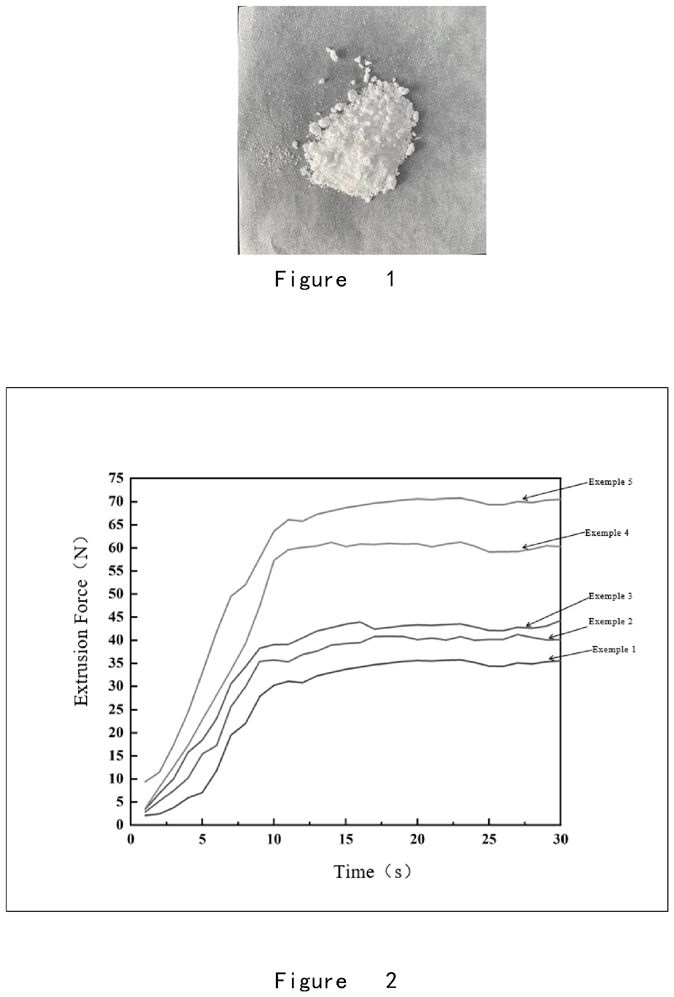

The obtained gel was added dropwise into 300 mL of acetone for washing, BDBE was eluted while the gel was solidified. The solidified product was collected by filtration and washed with absolute ethanol. After washing, approximately 8 g of a solid powder of hydroxyapatite-hyaluronic acid-hydroxypropyl methylcellulose gel was obtained by freeze-drying, as shown in Figure 1.

1 g of the solid powder was taken and reconstituted in 11.5 g of phosphate buffer solution to obtain a homogeneous hydroxyapatite-hyaluronic acid-hydroxypropyl methylcellulose gel.

In the reconstituted gel, the concentration of hyaluronic acid (excluding hydroxyapatite) is 2 w/w % (20 mg/g), the concentration of hollow hydroxyapatite microspheres is 5 w/w % (50 mg/g), and the pH is approximately 7.

### Example 2: Preparation of a Biocompatible Solid Particle Gel with Different Association Degrees (HA:HPMC=2:1)

2 g of hyaluronic acid and 1 g of hydroxypropyl methylcellulose were added to 27 g of water, and mixed at 500 rpm at room temperature until homogeneous. Subsequently, 1 g of sodium hydroxide (1 M) solution was added to the mixture to adjust the pH > 12, and mixed further at 500 rpm for 30 min. 5.26 g of hollow hydroxyapatite microspheres with a particle size of 20~50 µm was added to the mixture, and mixed again at 300 rpm for 30 min. Subsequently, 0.2 g of 1,4-butanediol diglycidyl ether (BDDE) was added, and the mixture was homogenized at 300 rpm for another 30 min. The homogenized mixture was placed in a 45°C oven for 3 h, and then let it stand at 25°C for 12 h. Then, 5 g of hydrochloric acid (0.5 M) was added to adjust the pH < 7 to terminate the crosslinking reaction. The mixture was stirred at 300 rpm for 10 minutes to obtain a gel-like, slightly acidic composite gel in a state between solid and liquid, containing hydroxyapatite hollow microspheres.

The obtained gel was added dropwise into 300 mL of acetone for washing, BDDE was eluted while the gel was solidified. The solidified product was collected by filtration and washed with absolute ethanol. After washing, approximately 8 g of a solid powder of hydroxyapatite-hyaluronic acid-hydroxypropyl methylcellulose gel was obtained by freeze-drying.

1 g of the solid powder was taken and reconstituted in 11.5 g of phosphate buffer solution to obtain a homogeneous hydroxyapatite-hyaluronic acid-hydroxypropyl methylcellulose gel.

In the reconstituted gel, the concentration of hyaluronic acid (excluding hydroxyapatite) is 2 w/w % (20 mg/g), the concentration of hollow hydroxyapatite microspheres is 5 w/w % (50 mg/g), and the pH is approximately 7.

Subsequently, 3.32 g of hollow hydroxyapatite microspheres with a particle size of 20~50 µm was added to the gel, and homogenized at 300 rpm for 30 min, to obtain a hyaluronic acid-hydroxypropyl methylcellulose gel containing hydroxyapatite with different association degrees (the concentration of hollow microspheres is 25 w/w %).

### Example 3: Preparation of a Biocompatible Solid Particle Gel with Different Association Degrees (HA:HPMC=1:1)

2 g of hyaluronic acid and 2 g of hydroxypropyl methylcellulose were added to 36 g of water, and mixed at 500 rpm at room temperature until uniform. Subsequently, 1 g of sodium hydroxide (1 M) solution was added to the mixture to adjust the pH > 12, and mixed further at 500 rpm for 30 min. 5.26 g of hollow hydroxyapatite microspheres with a particle size of 20~50 µm was added to the mixture, and mixed again at 300 rpm for 30 min. Subsequently, 0.2 g of 1,4-butanediol diglycidyl ether (BDDE) was added, and the mixture was homogenized at 300 rpm for another 30 min. The homogenized mixture was placed in a 45°C oven for 3 h, and then let it stand at 25°C for 12 h. Then, 5 g of hydrochloric acid (0.5 M) was added to adjust the pH < 7 to terminate the crosslinking reaction. The mixture was stirred at 300 rpm for 10 minutes to obtain a gel-like, slightly acidic composite gel in a state between solid and liquid, containing hydroxyapatite hollow microspheres.

The obtained gel was added dropwise into 300 mL of acetone for washing, BDDE was eluted while the gel was solidified. The solidified product was collected by filtration and washed with absolute ethanol. After washing, approximately 9 g of a solid powder of hydroxyapatite-hyaluronic acid-hydroxypropyl methylcellulose gel was obtained by freeze-drying.

1 g of the solid powder was taken and reconstituted in 10.1 g of phosphate buffer solution to obtain a homogeneous hydroxyapatite-hyaluronic acid-hydroxypropyl methylcellulose gel.

In the reconstituted gel, the concentration of hyaluronic acid (excluding hydroxyapatite) is 2 w/w % (20 mg/g), the concentration of hollow hydroxyapatite microspheres is 5 w/w % (50 mg/g), and the pH is approximately 7.

Subsequently, 2.94 g of hollow hydroxyapatite microspheres with a particle size of 20~50 µm was added to the gel, and homogenized at 300 rpm for 30 min, to obtain a hyaluronic acid-hydroxypropyl methylcellulose gel containing hydroxyapatite with different association degrees (the concentration of hollow microspheres is 25 w/w %).

### Example 4: Preparation of a Biocompatible Solid Particle Gel with Different Association Degrees (HA:HPMC=1:2)

2 g of hyaluronic acid and 4 g of hydroxypropyl methylcellulose were added to 44 g of water, and mixed at 500 rpm at room temperature until homogeneous. Subsequently, 1 g of sodium hydroxide (1 M) solution was added to the mixture to adjust the pH > 12, and mixed further at 500 rpm for 30 min. 5.26 g of hollow hydroxyapatite microspheres with a particle size of 20~50 µm was added to the mixture, and mixed again at 300 rpm for 30 min. Subsequently, 0.2 g of 1,4-butanediol diglycidyl ether (BDDE) was added, and the mixture was homogenized at 300 rpm for another 30 min. The homogenized mixture was placed in a 45°C oven for 3 h, and then let it stand at 25°C for 12 h. Then, 5 g of hydrochloric acid (0.5 M) was added to adjust the pH < 7 to terminate the crosslinking reaction. The mixture was stirred at 300 rpm for 10 minutes to obtain a gel-like, slightly acidic composite gel in a state between solid and liquid, containing hydroxyapatite hollow microspheres.

The obtained gel was dropwise into 300 mL of acetone for washing, BDDE was eluted while the gel was solidified. The solidified product was collected by filtration and washed with absolute ethanol. After washing, approximately 11 g of a solid powder of hydroxyapatite-hyaluronic acid-hydroxypropyl methylcellulose gel was obtained by freeze-drying.

1 g of the solid powder was taken and reconstituted in 9.1 g of phosphate buffer solution to obtain a homogeneous hydroxyapatite-hyaluronic acid-hydroxypropyl methylcellulose gel.

In the reconstituted gel, the concentration of hyaluronic acid (excluding hydroxyapatite) is 2 w/w % (20 mg/g), the concentration of hollow hydroxyapatite microspheres is 5 w/w % (50 mg/g), and the pH is approximately 7.

Subsequently, 2.74 g of hollow hydroxyapatite microspheres with a particle size of 20~50 µm was added to the gel, and homogenized at 300 rpm for 30 min, to obtain a hyaluronic acid-hydroxypropyl methylcellulose gel containing hydroxyapatite with different association degrees (the concentration of hollow microspheres is 25 w/w %).

### Example 5: Preparation of a Biocompatible Solid Particle Gel with Different Association Degrees (HA:HPMC=1:3)

2 g of hyaluronic acid and 6 g of hydroxypropyl methylcellulose were added to 44 g of water, and mixed at 500 rpm at room temperature until homogeneous. Subsequently, 1 g of sodium hydroxide (1 M) solution was added to the mixture to adjust the pH > 12, and mixed further at 500 rpm for 30 min. 5.26 g of hollow hydroxyapatite microspheres with a particle size of 20~50 µm was added to the mixture, and mixed again at 300 rpm for 30 min. Subsequently, 0.2 g of 1,4-butanediol diglycidyl ether (BDDE) was added, and the mixture was homogenized at 300 rpm for another 30 min. The homogenized mixture was placed in a 45°C oven for 3 h, and then let it stand at 25°C for 12 h. Then, 5 g of hydrochloric acid (0.5 M) was added to adjust the pH < 7 to terminate the crosslinking reaction. The mixture was stirred at 300 rpm for 10 minutes to obtain a gel-like, slightly acidic composite gel in a state between solid and liquid, containing hydroxyapatite hollow microspheres.

The obtained gel was added dropwise into 300 mL of acetone for washing, BDDE was eluted and the gel was solidified. The solidified product was collected by filtration and washed with absolute ethanol. After washing, approximately 13 g of a solid powder of hydroxyapatite-hyaluronic acid-hydroxypropyl methylcellulose gel was obtained by freeze-drying.

1 g of the solid powder was taken and reconstituted in 6.1 g of phosphate buffer solution to obtain a homogeneous hydroxyapatite-hyaluronic acid-hydroxypropyl methylcellulose gel.

In the reconstituted gel, the concentration of hyaluronic acid (excluding hydroxyapatite) is 2 w/w % (20 mg/g), the concentration of hollow hydroxyapatite microspheres is 5 w/w % (50 mg/g), and the pH is approximately 7.

Subsequently, 1.84 g of hollow hydroxyapatite microspheres with a particle size of 20~50 µm was added to the gel, and homogenized at 300 rpm for 30 min, to obtain a hyaluronic acid-hydroxypropyl methylcellulose gel containing hydroxyapatite with different association degrees (the concentration of hollow microspheres is 25 w/w %).

### Comparative Example C1: Comparison of Association Strength of Hollow Hydroxyapatite Microspheres Added at Different Steps

Sample C1 (product prepared according to Example 1): The experimental method is the same as that in Example 4, with the difference being: only 5.26 g of hollow hydroxyapatite microspheres was added before the crosslinking reaction.

### Comparative Example C2: Comparison of Association Strength of Hollow Hydroxyapatite Microspheres Added at Different Steps

Sample C2: The experimental method is the same as that in Example 4, with the difference being: no hollow hydroxyapatite microspheres were added before the crosslinking reaction; only 5.26 g of hollow hydroxyapatite microspheres was added after reconstitution.

The association strength of the hollow hydroxyapatite microspheres in the different samples were compared.

### Comparative Example C3: Preparation of a Gel without Cellulose Polysaccharide Polymer

2 g of hyaluronic acid was added to 18 g of water, and mixed at 500 rpm at room temperature until homogeneous. Subsequently, 1 g of sodium hydroxide (1 M) solution was added to the mixture to adjust the pH > 12, and mixed further at 500 rpm for 30 min. 5.26 g of hollow hydroxyapatite microspheres with a particle size of 20~50 µm was added to the mixture, and mixed again at 300 rpm for 30 min. Subsequently, 0.2 g of 1,4-butanediol diglycidyl ether (BDDE) was added, and the mixture was homogenized at 300 rpm for another 30 min. The homogenized mixture was placed in a 45°C oven for 3 h, and then let it stand at 25°C for 12 h. Then, 5 g of hydrochloric acid (0.5 M) was added to adjust the pH < 7 to terminate the crosslinking reaction. The mixture was stirred at 300 rpm for 10 minutes to obtain a gel-like, slightly acidic composite gel in a state between solid and liquid, containing hydroxyapatite hollow microspheres.

The obtained gel was added dropwise into 300 mL of acetone for washing. During this process, BDDE is eluted while the gel was solidified. The solidified product was collected by filteration and washed with absolute ethanol. After washing, approximately 7 g of a solid powder of hydroxyapatite-hyaluronic acid gel was obtained by freeze-drying.

1 g of the solid powder was taken and reconstituted in 13.25 g of phosphate buffer solution to obtain a homogeneous hydroxyapatite-hyaluronic acid gel.

In the reconstituted gel, the concentration of hyaluronic acid (excluding hydroxyapatite) is 2 w/w % (20 mg/g), the concentration of hollow hydroxyapatite microspheres is 5 w/w % (50 mg/g), and the pH is approximately 7.

As shown in Table 1, the addition of the first and second biocompatible solid particles in the comparative examples is presented.

**Table 1. Biocompatible Solid Particles Added in Comparative Examples C1-C3**

| Comparative Example | First biocompatible solid particles | Second biocompatible solid particles |
|---|---|---|
| C1 | 5.26 g | No addition |
| C2 | No addition | 5.26 g |
| C3 | 5.26 g | No addition |

### Test 1 - Injectability Measurement

The syringe plunger was pushed at a constant speed (30 mm/min). A hypodermic needle (27G) was attached during the experiment to simulate actual injection conditions. As the plunger was advanced at a constant speed, the sample in the syringe was extruded through the needle, thereby generating an extrusion force curve. The extrusion force curve reveals the variation in extrusion force during the sample extrusion process. It is easy to extrude the sample under a low extrusion force; while it is difficult to extrude the sample under a high extrusion force. A large fluctuation in the extrusion force indicates that there is an uneven distribution or aggregation/concentration phenomenon in the sample.

As shown in Figure 2, the results indicate that: none of the five sample groups from Examples 1, 2, 3, 4, and 5 showed signs of uneven dispersion or aggregation/concentration. Furthermore, as the proportion of HPMC increased, the samples became progressively more difficult to be extruded.

### Test 2 - Association Strength of Hydroxyapatite

### Method 1

30 g of the reconstituted gel was accurately weighed using an electronic balance and placed in a test tube. The tube was left standing at room temperature for one week, or even longer, to observe the sedimentation of hydroxyapatite.

The products obtained from Example 1 and C3 were evaluated using the above method to assess the capacity of different gels to carry hydroxyapatite. As shown in Figure 3, significant sedimentation of nano-cluster hollow hydroxyapatite microspheres was observed in C3.

The results indicate that: the incorporation of HPMC can enhance the capacity of the gel to carry hollow hydroxyapatite microspheres.

### Method 2

1.5 g of the reconstituted gel was accurately weighed using an electronic balance and placed in a 2 mL Eppendorf tube. Tubes were placed symmetrically in an Eppendorf centrifuge and centrifuged at 3000 rpm (735 × g force) for 10 min. After centrifugation, two phases were observed. The bottom phase in the centrifuge tube contained hydroxyapatite particles separated from the gel, and the upper phase contained the remaining gel portion, which still had particles attached to it. These two phases were separated. The gel in the upper phase was digested using hyaluronidase, and the hydroxyapatite particles were precipitated by centrifugation, washed with water, dried, and weighed. The hydroxyapatite particles in the bottom phase were dried and weighed. The percentage of hydroxyapatite in each phase was then calculated to reflect the association strength of hydroxyapatite.

**Table 2. Percentage of Hydroxyapatite in Homogeneous Gels Made with Different Ratios of HA to HPMC**

| Sample | | Total gel weight (g) | Dry weight of HAP (g) | Percentage of HAP in total gel (%) | Total HAP content (%) |
|---|---|---|---|---|---|
| Example 2 | Upper phase | 2.1047 | 0.3692 | 17.54 | 25.07 |
| | Bottom phase | | 0.1585 | 7.53 | |
| Example 3 | Upper phase | 2.0918 | 0.4338 | 20.74 | 24.83 |
| | Bottom phase | | 0.0856 | 4.09 | |
| Example 4 | Upper phase | 1.9811 | 0.4244 | 21.42 | 24.16 |
| | Bottom phase | | 0.0543 | 2.74 | |
| Example 5 | Upper phase | 2.1609 | 0.1182 | 5.47 | 25.28 |
| | Bottom phase | | 0.4178 | 19.81 | |
| Sample C1 | Upper phase | 2.2433 | 0.0402 | 1.79 | 5.08 |
| | Bottom phase | | 0.0738 | 3.29 | |
| Sample C2 | Upper phase | 1.9874 | 0.0010 | 0.05 | 5.14 |
| | Bottom phase | | 0.1012 | 5.09 | |

The centrifugation results are shown in Table 2. The slight deviation between the loading of hollow hydroxyapatite microspheres in Examples 2-5 and the theoretical 25 w/w% loading, as well as the slight deviation between the loading of hollow hydroxyapatite microspheres in samples C1 and C2 and the theoretical 5 w/w% loading, can all be attributed to weighing errors.

The results indicate that after centrifugation of the homogeneous gel compositions from Example 2, Example 3, and Example 4, the HAP remaining in the upper phase still accounted for 69.96%, 83.53%, and 88.66% of the total dry weight of HAP in the homogeneous gel composition, respectively. In contrast, for samples C1 and C2 after centrifugation, the HAP in the upper phase accounted for only 35.24% and 0.97% of the total dry weight of HAP in the homogeneous gel composition, respectively. For sample C2 after centrifugation, almost all particles (>99%) were separated from the gel. Among them, when the HA to HPMC ratio was 1:2 in Example 4, the hollow hydroxyapatite microspheres exhibited the optimal association strength.

The samples obtained from Example 1, Example 2, Example 3, Example 4, and Example 5 were subjected to filling, sterilization, and an accelerated stability study. The accelerated test storage conditions were: temperature 50°C ± 2°C, humidity 75% ± 5%.

### Test 3 - Average Extrusion Force Test

The syringe plunger was pushed at a constant speed (30 mm/min). A hypodermic needle (27G) was attached during the experiment to simulate actual injection conditions. As the plunger was advanced at a constant speed, the sample in the syringe was extruded through the needle, thereby generating an extrusion force curve. The average extrusion force value in the plateau region of the extrusion force curve was recorded.

The results of the average extrusion force are shown in Table 3. The results indicate that after three months of the accelerated stability study, the average extrusion force of the samples showed no significant change.

**Table 3. Average Extrusion Force Record Table for Samples in the Accelerated Stability Study**

| | Average extrusion force at month 0 (N) | Average extrusion force at month 1 (N) | Average extrusion force at month 3 (N) |
|---|---|---|---|
| Example 1 | 33.78 | 34.57 | 33.26 |
| Example 2 | 38.60 | 38.50 | 38.64 |
| Example 3 | 41.28 | 41.22 | 40.85 |
| Example 4 | 61.85 | 60.74 | 61.03 |
| Example 5 | 70.14 | 68.77 | 69.05 |

### Test 4 - Hydroxyapatite Sedimentation

Method: The samples were placed under accelerated test storage conditions, and the sedimentation of hydroxyapatite was periodically observed.

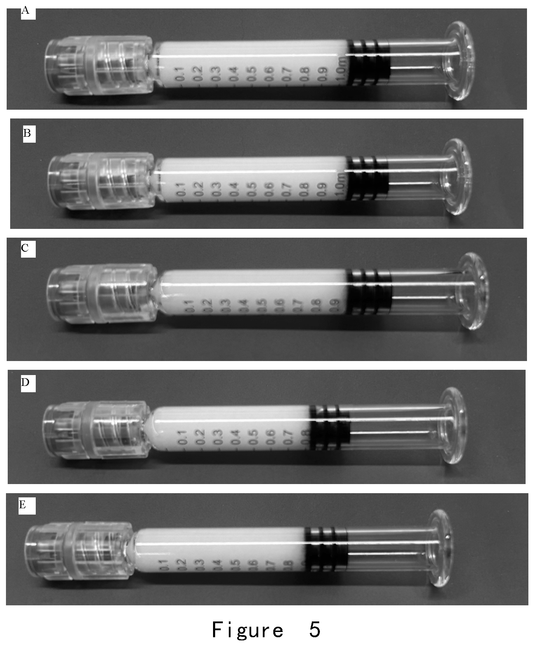

The hydroxyapatite sedimentation results are shown in Table 4. Real photographs are presented in Figure 5. The results indicate that after three months of the accelerated stability study, the samples showed no significant sedimentation of hydroxyapatite.

**Table 4. Association Strength Record Table of Hydroxyapatite in Accelerated Test Samples**

| Example | Result description |
|---|---|
| Example 1 | No sedimentation |
| Example 2 | No sedimentation |
| Example 3 | No sedimentation |
| Example 4 | No sedimentation |
| Example 5 | No sedimentation |

### Discussion

The craniofacial skeleton forms the foundation of human facial aesthetic features; therefore, facial bone sculpting is a potential cosmetic procedure. Hydroxyapatite (HAP), as the main mineral component of bone tissue, exhibits good biocompatibility and bone conductivity properties, and has been widely used as a bone substitute for many years. Furthermore, Youngmin H et al. incorporated 0.5-1 w/w% of HAP into a fructan-based hydrogel as a long-lasting dermal filler to enhance collagen production *in vivo*, thereby improving the wrinkle-smoothing effect of the filler. Experimental results also indicate that the addition of HAP promotes the proliferation of human dermal fibroblasts, improves the in vivo stability of the filler, and stimulates collagen production. HAP can provide dermal support and promote local collagen regeneration. However, its effect on stimulating collagen regeneration is relatively slow-acting, leading to imperfect early-stage collagen formation at the filler site. This readily causes secondary migration of HAP particles, resulting in suboptimal clinical outcomes and necessitating multiple corrective procedures. Currently, researchers have developed various polymer/HAP composite systems, where polymer adhesives include collagen, alginate, chitosan, carboxymethyl chitin, carboxymethylcellulose, polyhydroxybutyrate, hyaluronic acid, etc. Nevertheless, it remains challenging to solve the problem of secondary dispersion of HAP particles within the composite system.

The gel material and the injectable homogeneous gel composition of the present invention contain a composite structure composed of specific components. In this structure, hyaluronic acid, a cellulose polysaccharide polymer, and the first biocompatible solid particles form a first mixture. In the presence of a crosslinking agent, the hyaluronic acid in the first mixture undergoes a crosslinking reaction, thereby forming a crosslinked mixture. The mixture of crosslinked component (Z2) hyaluronic acid and component (Z3) cellulose-based polysaccharide polymer serves as a gel support skeleton for component (Z1) the first biocompatible solid particles, endowing the first biocompatible solid particles with a high degree of association and high loading capacity. In the injectable homogeneous gel composition of the present invention, the gel material of the present invention and the second biocompatible solid particles are combined through adsorption and physical mixing, thereby endowing the second biocompatible solid particles with a low degree of association. This ultimately forms a homogeneous gel composition characterized by both high and low association degrees. Tests have shown that the homogeneous gel composition of the present invention contains at least two types of biocompatible solid particles with different association degrees. This enables the gradual release of the biocompatible solid particles from the matrix, allowing them to stably exist in the composite system over a long term.

All documents mentioned in the present invention are incorporated herein by reference as if each document were individually incorporated by reference. Furthermore, it should be understood that after reading the above teachings of the present invention, those skilled in the art can make various changes or modifications to the present invention, and these equivalent forms also fall within the scope defined by the appended claims of this application.

## Claims

1. A gel material, **characterized in that** the gel material comprises following components:
(Z1) the first biocompatible solid particles;
(Z2) hyaluronic acid (HA); and
(Z3) a cellulose-based polysaccharide polymer;
wherein the gel material is in a solid powdery form or a gel form.

2. The gel material according to claim 1, **characterized in that** the gel material in a gel form possesses physicochemical characteristics selected from the group consisting of:
(a) the gel material in a gel form comprises highly associated biocompatible solid particles;
(b) the gel material in a gel form has a pH of 6-8;
(c) the gel material in a gel form has a water content of 75%-95%.

3. The gel material according to claim 1, **characterized in that** the gel material in a solid powdery form is formed by drying and crushing the gel material in a gel form.

4. The gel material according to claim 1, **characterized in that** the hyaluronic acid and the cellulose-based polysaccharide polymer in the gel material form a mixture, and the hyaluronic acid undergoes crosslinking to form a gel composite support skeleton.

5. The gel material according to claim 1, **characterized in that** component (Z1) the first biocompatible solid particles and the gel composite support skeleton form a composite structure.

6. The gel material according to claim 1, **characterized in that** the first biocompatible solid particles are selected from the group consisting of: calcium phosphate salt particles, silicate particles, calcium sulfate salt particles, ceramic particles, biological bone matrix particles, organic solid particles, or combinations thereof.

7. The gel material according to claim 1, **characterized in that** the cellulose-based polysaccharide polymer is selected from the group consisting of: hydroxypropyl methylcellulose, carboxymethyl cellulose, hydroxyethyl cellulose, ethyl hydroxyethyl cellulose, sodium carboxymethyl cellulose, carboxymethyl hydroxyethyl cellulose, hydroxypropyl hydroxyethyl cellulose, methyl cellulose, methyl hydroxymethyl cellulose, methyl hydroxyethyl cellulose, carboxymethyl methyl cellulose, or combinations thereof.

8. A method for preparing the gel material according to claim 1, **characterized in that** it comprises the following steps:
(S1) providing the first mixture, the first mixture comprising: hyaluronic acid, a cellulose polysaccharide polymer, and the first biocompatible solid particles;
(S2) in the presence of a crosslinking agent, subjecting the hyaluronic acid in the first mixture to a crosslinking reaction, thereby forming a crosslinked mixture with the cellulose-based polysaccharide polymer.

9. The method according to claim 8, **characterized in that** the crosslinking agent is selected from the group consisting of: 1,4-Butanediol diglycidyl ether, poly(ethylene glycol) diglycidyl ether, 1,6-Hexanediol diglycidyl ether, polypropylene glycol diglycidyl ether, polytetramethylene glycol diglycidyl ether, polyglycerol polyglycidyl ether, neopentyl glycol diglycidyl ether, ethylene glycol diglycidyl ether, or combinations thereof.

10. An injectable homogeneous gel composition, **characterized in that** the homogeneous gel composition comprises:
(Y1) the second biocompatible solid particles; and
(Y2) the gel material according to claim 1.

11. The homogeneous gel composition according to claim 10, **characterized in that** (W1+W2)/W0 = 5:1-20:1; preferably 8:1-15:1; wherein W1 is the weight of the first biocompatible solid particles, W2 is the weight of the second biocompatible solid particles, and W0 is the weight of the reconstituted gel material (excluding the biocompatible solid particles).

12. The homogeneous gel composition according to claim 10, **characterized in that** the homogeneous gel composition has one or more characteristics selected from the group consisting of:
(a) the homogeneous gel composition comprises biocompatible solid particles of both high association degree and low association degree;
(b) the biocompatible solid particles of the homogeneous gel composition account for 2%-95%, preferably 3%-80%, more preferably 4%-60%, most preferably 5%-30% of the dry weight of the homogeneous gel composition;
(c) the hyaluronic acid content in the homogeneous gel composition is 0.5%-4% w/w (5-40 mg/g), calculated based on the weight of the homogeneous gel composition excluding the biocompatible solid particles;
(d) the cellulose-based polysaccharide polymer content in the homogeneous gel composition is 0.2%-8% w/w (2-80 mg/g), calculated based on the weight of the homogeneous gel composition excluding the biocompatible solid particles.

13. A method for preparing the homogeneous gel composition according to claim 10, **characterized in that** it comprises following steps:
(S3) providing the second mixture, the second mixture comprising: the gel material in a gel form according to claim 1 and the second biocompatible solid particles, or the gel material in a solid powdery form, the second biocompatible solid particles, and water or an aqueous buffer solution;
(S4) mixing the second mixture uniformly to form the homogeneous gel composition.

14. Use of the homogeneous gel composition according to claim 10, **characterized in that** the use of the homogeneous gel composition comprises: dermal filling, and bone sculpting.

15. A kit, **characterized in that** the kit comprising following components: the homogeneous gel material according to claim 1, or the homogeneous gel composition according to claim 10.
